# FASCICULE DE BREVET EUROPEEN

(11) **EP 2 582 394 B1**
(45) Date de publication et mention de la délivrance du brevet: **23.10.2019**
(21) Numéro de dépôt: 11735499.3
(22) Date de dépôt: 15.06.2011
(51) Int. Cl.: A61K 47/18, A61K 47/26, A61K 38/00, A61K 39/395

(54) **COMPOSITION D'IMMUNOGLOBULINES HUMAINES STABILISEE**
STABILISIERTE HUMANE IMMUNGLOBULINZUSAMMENSETZUNG
STABILISED HUMAN IMMUNOGLOBULIN COMPOSITION

(30) Priorité: 15.06.2010 FR 1054721
(43) Date de publication de la demande: 24.04.2013
(73) Titulaire: Laboratoire Français du Fractionnement et des Biotechnologies Société Anonyme, 91940 Les Ulis (FR)
(72) Inventeur: HUILLE, Sylvain, F-92160 Antony (FR); COHEN-TANNOUDJI, Laetitia, F-95014 Paris (FR)
(74) Mandataire: Chajmowicz, Marion
(86) Numéro de dépôt international: PCT/FR2011/051358
(87) Numéro de publication internationale: WO 2011/157950

(56) Documents cités:
- EP-A2- 0 196 761
- WO-A1-95/03826
- WO-A1-2005/073252
- WO-A2-98/44948
- WO-A2-03/039485
- FR-A1- 2 895 263
- US-A- 5 945 098
- US-A1- 2007 036 779

## Description

L'invention a trait à la formulation d'immunoglobulines G humaines, utiles en thérapie.

De nombreuses pathologies sont actuellement traitées par des compositions d'immunoglobulines G (IgG). On peut citer par exemple les déficits immunitaires primitifs avec défaut de production d'anticorps, la maladie de Kawasaki, le purpura thrombopénique immunologique de l'enfant et de l'adulte, les déficits immunitaires secondaires avec défaut de production d'anticorps, en particulier la leucémie lymphoïde chronique ou myélome associés à des infections à répétition, l'infection de l'enfant par le VIH associé à des infections bactériennes, les neuropathies motrices multifocales, le syndrome de Guillain-Barré, les infections aiguës sévères ou chroniques à Parvovirus B19, l'immunodéficience acquise ou constitutionnelle, la dermatomyosite cortico-résistante, la myasthénie aiguë, la polyradiculonévrite chronique idiopathique, le purpura thrombopénique immunologique, par exemple associé à l'infection par le VIH, le syndrome de l'homme raide (Stiffman syndrome), la neutropénie auto-immune, l'erythroblastopénie auto-immune résistante, le syndrome d'anti-coagulation acquise par auto-anticorps, la polyarthrite rhumatoïde, etc.

Au cours de ces dernières années, la très forte demande d'IgG a engendré des situations de tension extrêmes sur les approvisionnements, pouvant aller jusqu'à des situations de pénurie en Europe et aux Etats Unis d'Amérique.

Dans ce contexte, il y a un besoin grandissant de produire des compositions d'IgG, injectables par voie intraveineuse, à partir par exemple de plasmas humains. Avec l'essor de ces besoins en IgG, la stabilisation de ces compositions d'IgG injectables par voie intraveineuse (IgGIV) en vue de leur utilisation thérapeutique et de leur conservation revêt un caractère fondamental.

A cet égard, on sait qu'il est nécessaire de stabiliser les IgGIV pour éviter notamment la formation d'agrégats (oligomères et polymères) susceptibles d'activer le système du complément avec des risques associés de réactions anaphylactiques, céphalées, fièvres, rougeurs, baisse de tension (Bolli et al, Biologicals, 2010, 38 :150-157). Par ailleurs, la présence de dimères dans les IgGIV a été corrélée à des baisses de pression artérielle *in vivo.* D'autres dégradations physicochimiques peuvent également intervenir au cours de la conservation des IgG comme, entre autres, l'oxydation et l'hydrolyse.

La stabilisation des IgG nécessite donc l'ajout de composés, classiquement choisis parmi les sucres et les acides aminés, afin d'obtenir non seulement des compositions d'IgG non dégradées appropriées à un usage thérapeutique mais également des compositions d'IgG présentant une stabilité accrue durant le stockage.

Plusieurs formulations d'immunoglobulines humaines destinées à une administration intraveineuse ont été proposées (cf notamment le brevet US 5,945,098, les demandes de brevets WO2005/049078 ou WO96/07429). Une formulation particulièrement efficace pour stabiliser les compositions d'immunoglobulines est décrite dans la demande de brevet internationale WO 2004/091656 déposée par la Demanderesse. Cette demande de brevet divulgue une composition contenant 50 g/l d'IgG, 50 g/l de mannitol, 10 g/l de glycine et 50 ppm de détergent, 50 ppm de détergent correspondant à une concentration de 50 mg/l de détergent.

Des compositions d'IgGIV lyophilisées sont disponibles dans le commerce, par exemple sous les noms de marques Polygam™ (American Red Cross), Gammar IV™ (Armour Pharmaceutical Company) et Venoglobulin™I (Alpha) contenant comme stabilisants du glucose à 2%, du saccharose à 5% et du D-mannitol à 2% respectivement.

Des compositions liquides d'IgGIV qui contiennent comme stabilisants du maltose à 10%, de la glycine de 0,16 à 0,24 M et du D-sorbitol à 5% sont respectivement connues sous les noms de marque Octagam™, (Octapharma), Gamunex™ 10% (Talecris) et Venoglobulin™ (Alpha).

Cependant il existe toujours un besoin en des formulations d'IgIV qui soient bien tolérées et qui soient suffisamment stables pour une conservation optimale, facilitant leur utilisation.

### Résumé de l'invention :

La Demanderesse a maintenant mis au point une composition pharmaceutique comprenant des immunoglobulines G humaines formulées avec de la glycine et un détergent non ionique, à un pH inférieur ou égal à 4,8.

Les inventeurs ont plus particulièrement montré l'importance d'un faible pH pour stabiliser cette formulation.

Un objet de l'invention est donc une composition pharmaceutique comprenant des immunoglobulines G humaines (IgG), conforme à la revendication 1.

La composition présente un pH compris entre 4,4 et 4,8. De préférence le pH est de 4,6.

La composition selon l'invention est avantageusement sous forme liquide. Elle peut être préparée directement ou être obtenue par reconstitution avec de l'eau, à partir d'un lyophilisat.

Un autre objet de l'invention est une composition solide obtenue par dessication, de préférence lyophilisation, d'une composition liquide telle que définie ici.

### Description détaillée:

### Définitions

On entend par « Immunoglobulines G humaines » ou « IgG humaines » dans le cadre de l'invention, des immunoglobulines polyvalentes qui sont essentiellement des IgG, incluant éventuellement des IgM. Il peut s'agir d'immunoglobulines entières, ou de fragments tels que F(ab')2 ou F(ab) et toute fraction intermédiaire obtenue au cours du procédé de fabrication des immunoglobulines polyvalentes.

Le terme « stabilité » correspond à la stabilité physique et/ou chimique des IgG.

Le terme « stabilité physique » se réfère à la réduction ou l'absence de formation d'agrégats insolubles ou solubles des formes dimériques, oligomériques ou polymériques des Ig, ainsi qu'à la réduction ou l'absence de toute dénaturation structurale de la molécule.

Le terme "stabilité chimique" se réfère à la réduction ou l'absence de toute modification chimique des IgG pendant le stockage, à l'état solide ou sous forme dissoute, dans des conditions accélérées. Par exemple, les phénomènes d'hydrolyse, déamination, et/ou oxydation sont évités ou retardés. L'oxydation des acides aminés contenant du soufre est limitée.

### Formulations :

La concentration en IgG est de 100 g/l.

Les concentrations sont déterminées vis à vis des compositions sous forme liquide, avant dessication, ou après reconstitution sous forme de préparation injectable.

La composition ne contient pas de mannitol. En effet, il a été montré que le mannitol n'était pas essentiel à la stabilisation de la formulation.

Plus particulièrement, dans un mode de réalisation préféré, les seuls excipients sont la glycine et le détergent non-ionique.

Le détergent non ionique utilisé dans la composition selon l'invention est le polysorbate 80 (ou Tween®80 qui est du polyoxyéthylènesorbitanne-monooléate).

Les compositions de l'invention peuvent aussi comprendre d'autres additifs. Un tel additif peut aussi bien représenter un composé choisi parmi les différentes catégories de stabilisants classiquement utilisés dans le domaine technique de l'invention, tels que les tensioactifs, les sucres et les acides aminés, qu'un excipient ajouté à la formulation afin d'en ajuster, par exemple, le pH, la force ionique etc. Alternativement, la composition selon l'invention ne comprend pas d'autres excipients que lesdits glycine et détergent non ionique. Une telle composition présente l'avantage d'offrir une bonne stabilisation des compositions d'immunoglobulines et une réduction des durées et des coûts de préparation à l'échelle industrielle grâce à la présence d'un nombre minimal efficace d'excipients ainsi que la présence d'une quantité minimale efficace d'excipients.

La composition selon l'invention comprend :
- 100 g/l d'IgG
- 250 mM de glycine
- 50 mg/l de polysorbate 80.

Les immunoglobulines G sont généralement obtenues par fractionnement du plasma sanguin humain, et présentées dans un milieu aqueux. Le milieu aqueux est composé d'eau pour préparation injectable (eau PPI) pouvant contenir des excipients pharmaceutiquement acceptables et compatibles avec les IgG. Les compositions d'IgG peuvent au préalable subir des étapes spécifiques d'inactivation/élimination de virus, tel qu'un traitement solvant détergent, une pasteurisation et/ou une nanofiltration. La composition selon l'invention comprend des IgG qui peuvent être polyclonales ou monoclonales. Les IgG peuvent être isolées à partir du sang humain ou animal ou produites par d'autres moyens, par exemple par des techniques de biologie moléculaire, par exemple dans des systèmes cellulaires bien connus de l'homme du métier. La composition selon l'invention est particulièrement adaptée aux IgG hautement purifiées. Avantageusement, les IgG de la présente invention sont obtenues par fractionnement du plasma humain. Des méthodes de fractionnement préférées du plasma humain sont décrites par Cohn et al (J. Am. Chem. Soc., 68, 459, 1946), Kistler et al. (Vox Sang., 7, 1962, 414-424), Steinbuch et al (Rev. Franç. Et. Clin. et Biol., XIV, 1054, 1969) et dans la demande de brevet WO 94/9334. Une méthode de préparation d'une composition d'immunoglobulines G est également décrite dans la demande de brevet WO 02/092632.

Les compositions liquides selon l'invention peuvent subir une dessiccation pour obtenir une forme solide qui se conserve plus longtemps et est plus pratique pour le transport et la commercialisation. La dessiccation est un procédé d'élimination de l'eau à un stade poussé. Il s'agit d'une déshydratation visant à éliminer autant d'eau que possible. Ce phénomène peut être naturel ou forcé. Cette dessiccation peut être réalisée à l'aide des techniques de lyophilisation, d'atomisation et de cryoatomisation. Le mode préféré d'obtention de la forme solide de la composition à usage pharmaceutique selon l'invention est la lyophilisation. Les méthodes de lyophilisation sont bien connues de l'homme du métier, voir par exemple [Wang et al, Lyophilization and development of solid protein pharmaceuticals, International Journal of Pharmaceutics, Vol 203, p 1-60, 2000]. D'autres procédés appropriés pour réduire le degré d'humidité ou la teneur en eau de la composition sont envisageables. De préférence le degré d'humidité est inférieur ou égal à 3% en poids, de préférence inférieur ou égal à 2,5%, de préférence inférieur ou égal à 2%, de préférence inférieur ou égal à 1,5%.

La composition selon l'invention peut être avantageusement soumise à une méthode d'élimination ou d'inactivation des agents infectieux, par exemple par chauffage à sec du lyophilisat.

La composition solide selon l'invention, de préférence sous forme lyophilisée, peut être dissoute dans de l'eau pour préparations injectables (ou « water for injection ou WFI »), pour obtenir une formulation à usage thérapeutique.

### Voies d'administration :

La composition de l'invention est utile en thérapie, et notamment sous forme injectable, de préférence par voie intraveineuse. La composition est alors sous forme liquide.

### Légende des Figures

Les **Figures 1A** et **1B** représentent des histogrammes montrant les mesures de diffusion de la lumière en mode dynamique (Figure 1A) ou en mode statique (Figure 1B), sur des formulations d'immunoglobulines dans de la glycine, à diverses valeurs de pH.
La **Figure 2** est un graphe qui illustre les résultats d'un suivi de mesure de turbidité (DO à 400nm) de formulations d'immunoglobulines à divers pH, dans des conditions de stress thermique (57°C).
La **Figure 3** est un graphe qui illustre l'influence du pH sur la turbidité de formulations d'immunoglobulines, en conditions de stress d'agitation rotative (retournement des flacons).

### Exemples :

### Exemple 1 : Etude de l'influence du pH sur le comportement des immunoglobulines

L'influence du pH a été testée sur des formulations d'immunoglobulines G humaines concentrées à 10% dans un tampon glycine 100mM, le pH étant ajusté dans des conditions non dénaturantes, c'est-à-dire par dialyse contre un tampon ajusté en pH permettant d'atteindre le pH visé. Plusieurs pH sont testés : 4,6 ; 5,2 ; 5,7; 6,4 ; 6,8.

L'état d'agrégation des immunoglobulines est suivi par un test de diffusion de la lumière (angle de 90°), après ajustement du pH (t=0). Après ajustement et sans appliquer de stress sur les formulations, les solutions présentent des états d'agrégation différents.

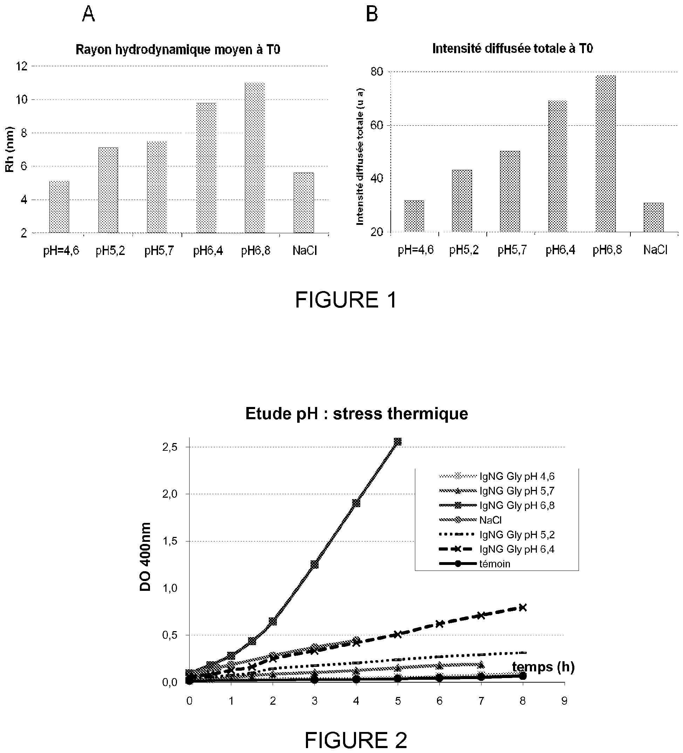

En effet, les mesures de diffusion de la lumière en mode statique et en mode dynamique montrent une augmentation de l'agrégation submicronique avec l'élévation du pH (**Figures 1A et 1B**).

La solution protéique est composée de différentes sous-classes d'immunoglobulines (Ig1, Ig2, Ig3 et Ig4) qui présentent une hétérogénéité pour leur point isoélectrique (pI), allant de 5 à 9 environ. Le pH jouerait en fait directement sur la charge effective des immunoglobulines (Ig), pour celles qui ont un pI bas (<7,0). Dans cette zone de pH, certaines Ig passent d'une charge globalement positive à une charge globalement négative, ce qui change la nature des interactions électrostatiques avec les autres Ig. L'agrégation observée ici après ajustement du pH s'apparente à de l'oligomérisation.

Les tests montrent que pour avoir des interactions globalement répulsives et donc stabilisantes, un pH d'environ 4,6 est favorable.

La **Figure 2** illustre les résultats d'un suivi de mesure de turbidité (DO à 400nm) dans des conditions de stress thermique (57°C) montrant l'influence du pH sur l'agrégation macroscopique des Ig. Là encore le pH de 4,6 est le plus favorable, l'élévation du pH favorisant les phénomènes d'agrégation.

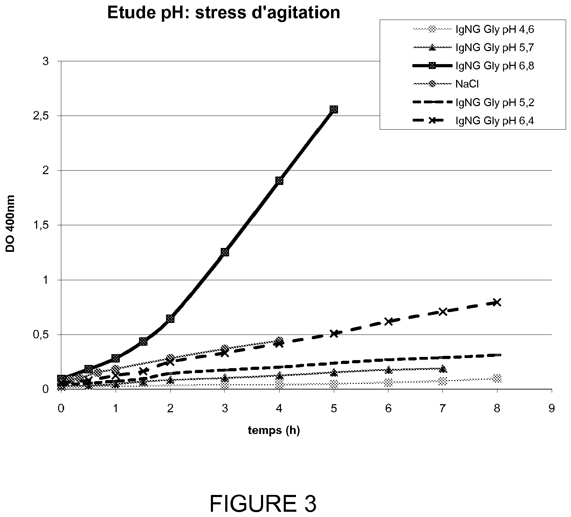

Un stress d'agitation rotative (retournement des flacons) est aussi conduit pour justifier le rôle du pH sur l'agrégation aux interfaces eau-air. Des mesures de turbidité sont réalisées après centrifugation et remise en suspension pour éliminer les microbulles éventuelles. La **Figure 3** montre que l'agrégation des Ig aux interfaces est fortement influencée par le pH.

### Exemple 2 : Formulations d'immunoglobulines

Plusieurs formulations d'immunoglobulines humaines à une concentration de 10% ont été préparées, avec les excipients suivants, à pH 4,6 :

**Tableau 1 : Formulations testées**

| | |
|---|---|
| **IgNG** | **Glycine** (93mM) - **Mannitol** (175mM- 32g/L) - **Tween 80** 50ppm |
| **GL** | **Glycine** (200mM) - **Leucine** (50mM) |
| **GT20** | **Glycine** (250mM) - **Tween 20** 20ppm |
| **GLT20** | **Glycine** (200mM) - **Leucine** (50mM)-**Tween 20** 5ppm |
| **GLM** | **Glycine** (100mM) - **Leucine** (50mM)-**Mannitol** (100mM-18g/L) |
| **GMT20** | **Glycine** (150mM) - **Mannitol** (100mM- 18g/L) - **Tween 20** 20ppm |
| **GT80** | **Glycine** (250mM) - **Tween 80** 50ppm |

Les immunoglobulines utilisées proviennent d'une solution concentrée à 168g/L à pH=4,7, sans aucun excipient de formulation, cette solution ayant été obtenue à partir d'un fractionnement de plasma humain, puis ultrafiltration tangentielle. Les formulations GT80 et GT20 ont été préparées par dilution des Ig dans des tampons de formulation pour atteindre un titre protéique de 100g/l, et les concentrations visées en excipients. De l'acide chlorhydrique a été utilisé pour ajuster le pH.

Les formulations ont été soumises à des tests de stabilité dite « accélérée », en les conservant à 25°C ou à 40°C, pendant 6, 13 ou 19 semaines.

La dégradation physique a été suivie, par analyse des phénomènes d'agrégation par HPSEC pour le suivi des dimères/oligomèes et polymères, la DLS (pour « dynamic light scattering », mesure de diffusion de la lumière) pour l'agrégation submicronique, le comptage de particules sub-visibles (taille entre 10 et 50µm), et observation visuelle (taille >50µm).

La stabilité chimique a été suivie par HPSEC et SDS-PAGE pour suivre la fragmentation, et le dosage des anti-HBs (anticorps anti-antigènes de surface de l'hépatite B) a fourni un indicateur de la stabilité de la fonction Fab.

L'activité anti-complémentaire (AAC) a été déterminée, par mesure de la captation aspécifique du complément par les Ig. Ce test décrit l'aptitude des immunoglobulines à activer le système du complément, une activation trop puissante du complément pouvant nuire à la tolérance du produit lors de son injection.

Les formulations IgNG, GT80 et GT20 ont globalement donné les meilleurs résultats.

Le tensioactif (Tween) a favorisé la stabilité physique des formulations.

De manière surprenante, la présence de mannitol ne s'est pas avérée essentielle, les formulations GT80 et GT20 (dépourvues de mannitol) présentant des stabilités au moins aussi bonnes que la formulation IgNG (avec mannitol).

Les formulations suivantes sont donc retenues comme les plus avantageuses :
- Formulation GT80: Glycine 250mM, Tween® 80 50ppm, pH =4,6
- Formulation GT20: Glycine 250mM, Tween® 20 20ppm, pH=4,6

### Exemple 3 : Stabilité de la formulation GT80 (pH=4,6)

La stabilité de la formulation GT80 est comparée à celle d'une formulation IgNG, pendant 12 mois à 25°C et à 40°C.

**Tableau 2 : Formulations GT80 et IgNG (10% d'immunoglobulines humaines IgIV)**

| | |
|---|---|
| **IgNG** | **Glycine** (93mM) - **Mannitol** (175mM- 32g/L) - **Tween 80** 50ppm |
| **GT80** | **Glycine** (250mM) - **Tween 80** 50ppm |

A T0, les flacons sont placés dans des armoires thermostatées à 25°C et 40°C, et leur stabilité selon le protocole de stabilité accélérée est suivie selon l'échéancier suivant :

**Tableau 3 : Echéancier de la stabilité accélérée**

| | **T0** | **T6S** | **T13S** | **T19S** | **T6,5M** | **T9M** | **T12M** |
|---|---|---|---|---|---|---|---|
| **25°C** | √ | √ | √ | √ | √ | √ | √ |
| **40°C** | | √ | √ | √* | | | √* |

| | | | | | | | |
|---|---|---|---|---|---|---|---|
| * analyses les plus discriminantes uniquement | | | | | | | |

Après 12 mois à 25°C et 40°C, les formulations GT80 et IgNG présentent une stabilité comparable :
- Les formulations mises en stabilité ont des comportements identiques du point de vue de la dégradation chimique : la fragmentation est observée à 40°C par HPSEC et SDS-PAGE et une perte de l'activité Fab à 40°C ;
- Les formulations sont identiques en ce qui concerne l'agrégation submicronique observée à 40°C en DLS et en HPSEC ;
- Une agrégation macroscopique est observée à 40°C ainsi que l'apparition d'une coloration jaune. Cette coloration est identique pour les formulations GT80 et IgNG. A 25°C, les 2 formulations sont incolores.
- Les écarts observés en AAC restent faibles. Les deux formulations évoluent de façon comparable à 25°C et 40°C. Le mannitol ne contribue pas à la stabilité d'IgNG vis-à-vis du test AAC.

Ces résultats de stabilité à 12 mois confirment que l'adjonction de Mannitol est sans effet sur la stabilité d'IgNG.

En définitive, la formulation GT80 constituée de Glycine (250 mM) et de Tween 80 (50 ppm) est une formulation stable adaptée à une utilisation thérapeutique commerciale. Après 12 mois à 25°C, toutes les analyses réalisées sont conformes selon la Pharmacopée Européenne.

## Revendications

1. Composition pharmaceutique liquide comprenant 100 g/l d'immunoglobulines G humaines (IgG), 250 mM de glycine, et 50 mg/l de Polysorbate 80, ladite composition présentant un pH compris entre 4,4 et 4,8, et ne contenant pas de mannitol.

2. Composition pharmaceutique selon la revendication 1, ladite composition présentant un pH de 4,6.

3. Composition selon l'une des revendications 1 à 2, **caractérisée en ce que** les seuls excipients sont la glycine et le détergent non-ionique.

4. Composition selon l'une des revendications 1 à 3, **caractérisée en ce que** les immunoglobulines G sont obtenues par fractionnement du plasma sanguin humain.

5. Composition selon l'une des revendications 1 à 4 qui est obtenue par reconstitution avec de l'eau, à partir d'un lyophilisat.

6. Composition solide obtenue par dessication, de préférence lyophilisation, d'une composition liquide selon l'une des revendications 1 à 5.

## Patentansprüche

1. Flüssige pharmazeutische Zusammensetzung umfassend 100 g/l humane Immunglobuline G (IgG), 250 mM Glycin und 50 mg/l Polysorbat 80, wobei besagte Zusammensetzung einen pH zwischen 4,4 und 4,8 aufweist und kein Mannit enthält.

2. Pharmazeutische Zusammensetzung gemäß Anspruch 1, wobei besagte Zusammensetzung einen pH von 4,6 aufweist.

3. Zusammensetzung gemäß einem der Ansprüche 1 bis 2, **dadurch gekennzeichnet, dass** die einzigen Exzipienten Glycin und das nichtionische Tensid sind.

4. Zusammensetzung gemäß einem der Ansprüche 1 bis 3, **dadurch gekennzeichnet, dass** die Immunglobuline G durch Fraktionierung des humanen Blutplasmas erhalten werden.

5. Zusammensetzung gemäß einem der Ansprüche 1 bis 4, die durch Rekonstitution eines Lyophilisats mit Wasser erhalten wird.

6. Feste Zusammensetzung, die durch Trocknung, vorzugsweise Lyophilisierung, einer flüssigen Zusammensetzung gemäß einem der Ansprüche 1 bis 5 erhalten wird.

## Claims

1. Liquid pharmaceutical composition which comprises 100 g/l of human immunoglobulins G (IgG), 250 mM of glycine, and 50 mg/l of Polysorbate 80, said composition having a pH between 4.4 and 4.8, and does not contain mannitol.

2. Pharmaceutical composition according to claim 1, said composition having a pH of 4.6.

3. Composition according to one of claims 1 to 2, **characterized in that** the only excipients are glycine and the non-ionic detergent.

4. Composition according to one of claims 1 to 3, **characterized in that** the immunoglobulins G are obtained by fractionation of human blood plasma.

5. Composition according to one of claims 1 to 4 which is obtained by reconstitution with water, from a lyophilizate.

6. Solid composition obtained by desiccation, preferably freeze-drying, of a liquid composition according to one of claims 1 to 5.
